# EUROPEAN PATENT APPLICATION

(11) **EP 2 950 483 A1**
(43) Date of publication of application: **02.12.2015**
(21) Application number: 15152269.5
(22) Date of filing: 23.01.2015
(51) Int. Cl.: H04L 12/28

(54) **IN-HOUSE CONTROL SYSTEM**

(30) Priority: 30.05.2014 JP 2014113417
(71) Applicant: Toshiba Lighting & Technology Corporation, Yokosuka-shi Kanagawa 237-8510 (JP)
(72) Inventor: Noguchi, Yoko, Kanagawa, 237-8510 (JP); Ide, Nagisa, Kanagawa, 237-8510 (JP)
(74) Representative: Willquist, Sofia Ellinor

(57) **Abstract**

According to one embodiment, an in-house control system is for controlling an in-house electric equipment. An acquisition unit acquires biological information of a user. A storage part stores the biological information acquired by the acquisition unit. A setting part sets schedule information. A control part controls the electric equipment based on the schedule information set by the setting part and the biological information stored in the storage part.

## Description

### FIELD

Embodiments described herein relate generally to an in-house control system for controlling an in-house electric equipment.

### BACKGROUND

Hitherto, a system such as a HEMS (Home Energy Management System) is known in which an in-house electric equipment and a home gateway are made communicable, and a user operates the electric equipment through the gateway by a personal computer or the like and can confirm the states and power use amounts of the electric equipment.

In such a system, the user can merely operate the electric equipment by the personal computer or the like, and there is no system in which an in-house electric equipment is automatically controlled so as to encourage a life in which the health of the user is promoted.

### BRIEF DESCRIPTION OF THE DRAWINGS

FIG. 1 is a conceptual view of an in-house control system of an embodiment.
FIG. 2 is a block diagram of a specific example of the in-house control system.
FIG. 3 is a timing chart of the control content of the in-house control system.
FIG. 4 is an explanatory view showing a display example in which the control content of the in-house control system is displayed on a smartphone.
FIG. 5 is a graph showing a relation between sleep depth and time with respect to sleep information used in the in-house control system.

### DETAILED DESCRIPTION

In general, according to one embodiment, an in-house control system is for controlling an in-house electric equipment. An acquisition unit acquires biological information of a user. A storage part stores the biological information acquired by the acquisition unit. A setting part sets schedule information by the user. A control part controls the electric equipment based on the schedule information set by the setting part and the biological information stored in the storage part.

According to the in-house control system, the in-house electric equipment is automatically controlled based on the schedule information and the stored biological information of the user, and the life in which the health of the user is promoted can be expected to be encouraged.

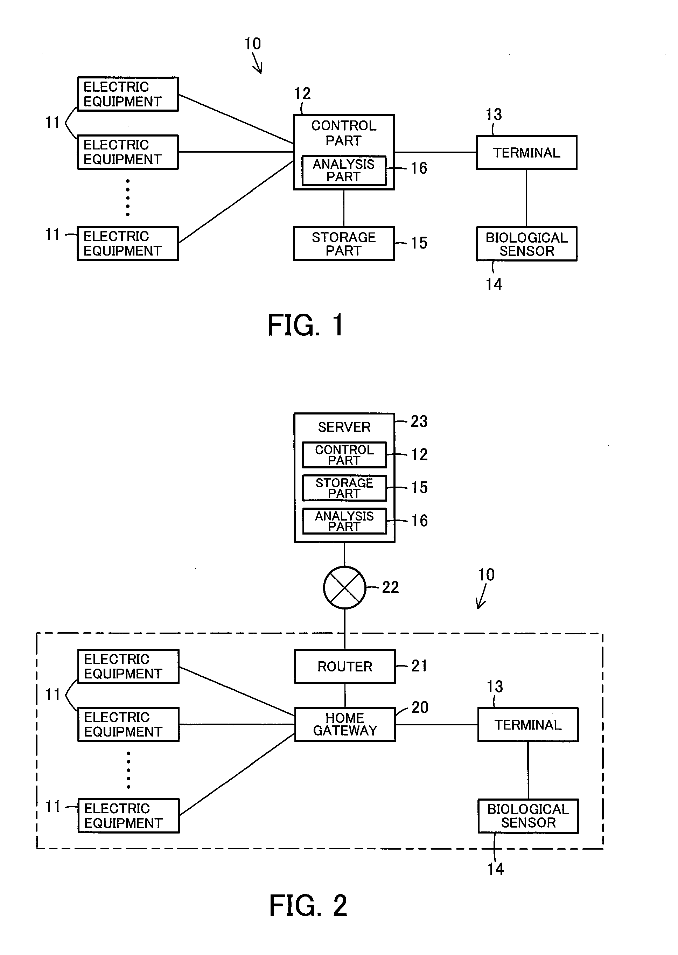

Hereinafter, an embodiment will be described with reference to FIG. 1 to FIG. 4.

FIG. 1 is a conceptual view of an in-house control system 10.

The in-house control system 10 controls plural electric equipments 11 arranged in a house, monitors the states and power use amounts of the electric equipments 11 and can display the information.

The in-house control system 10 includes a control part 12. The system includes the control part 12, the plural electric equipments 11, a terminal 13 as a setting part by which a user sets schedule information, a biological sensor 14 as an acquisition unit to acquire biological information of the user, a storage part 15 to store the biological information acquired by the biological sensor 14, and an analysis part 16 to statistically analyze data stored in the storage part 15.

The control part 12 controls the electric equipments 11 based on the schedule information set by the terminal 13 and the biological information stored in the storage part 15.

FIG. 2 is a block diagram of a specific example of the in-house control system 10.

The in-house control system 10 includes a home gateway 20 installed in the house. The plural electric equipments 11 and the terminal 13 are respectively communicably connected to the home gateway 20. Besides, when internet connection is made, the home gateway 20 is connected to internet 22 through a router 21, and the in-house control system 10 can access a server 23 connected to the internet 22.

The electric equipments 11 include a luminaire, a television, an air conditioner, a water heater and the like, which are installed in the house. The electric equipments 11 are mutually communicable with the home gateway 20 through a communication adopter. Either of wired and wireless communication systems may be used for the communication. When receiving control signals from the home gateway 20, the electric equipments 11 operate according to the control signals, or transmit state information to the home gateway 20. Among luminaires arranged in the house, at least a luminaire in a living room or a bedroom is of a color variable type in which light emission color can be changed between a daylight color and a bulb color and is dimmable.

The terminal 13 includes a personal computer, a smartphone, a tablet, a television or the like. The terminal 13 is mutually communicable with the home gateway 20. Either of wired and wireless communication systems may be used for the communication. The terminal 13 enables the user to set schedule information. With respect to the setting of the schedule information, the schedule information may be set by accessing the server 23 from the terminal 13, or the schedule information which is set by the terminal 13 may be transmitted to the server 23. Incidentally, the terminal 13 is also a display part to display information transmitted from the home gateway 20.

The biological sensor 14 is an activity meter capable of detecting biological information of the user, such as a pulse wave, breath and body movement. The biological sensor 14 can be always worn by the user, and is, for example, a bracelet-type sensor capable of being attached to the arm. The biological sensor 14 has a built-in battery, has a clock function and a memory function, and stores the detected biological information together with time information. The biological sensor 14 is communicable with, for example, the terminal 13. Either of wired and wireless communication systems may be used for the communication. The information stored in the biological sensor 14 is transmitted to the terminal 13, and is transmitted from the terminal 13 to the server 23.

The home gateway 20 is mutually communicable with the electric equipments 11, the terminal 13 and the server 23.

I n the embodiment shown in FIG. 2, the server 23 includes the control part 12, the storage part 15 and the analysis part 16. The home gateway 20 may have the function of the control part 12 to control the electric equipments 11.

Incidentally, instead of the server 23, the terminal 13 or the home gateway 20 may include the control part 12, the storage part 15 and the analysis part 16. I n this case, the in-house control system 10 may not be connected to the internet.

Next, the operation of the embodiment will be described.

The user wears the biological sensor 14 and lives. The biological sensor 14 detects the biological information of the user, such as the pulse wave, breath and body movement, and stores the biological information together with time information. The user periodically transfers the biological information stored in the biological sensor 14 to the terminal 13. The biological information is transmitted from the terminal 13 to the server 23, and the storage part 15 stores the biological information.

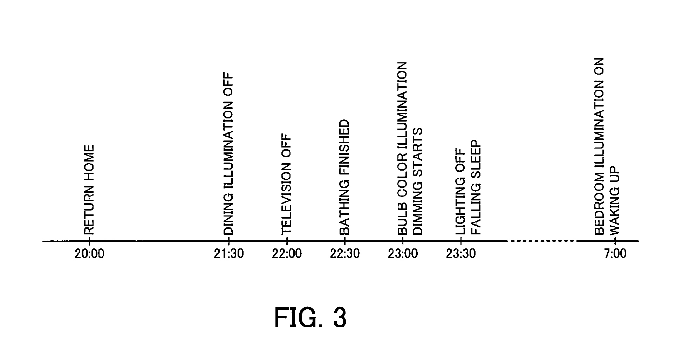

The operation of the in-house control system 10 will be described with reference to a timing chart of FIG. 3.

The user operates the terminal 13, and is assumed to set, as the schedule information, wake-up time on next day to "7:00". With respect to the setting of the schedule information, the schedule information may be set by accessing the server 23 from the terminal 13, or the schedule information which is set by the terminal 13 may be transmitted to the server 23.

The analysis part 16 of the server 23 calculates appropriate hypnagogic time based on the sleep information and daily activity amount information for the past one month stored in the storage part 15.

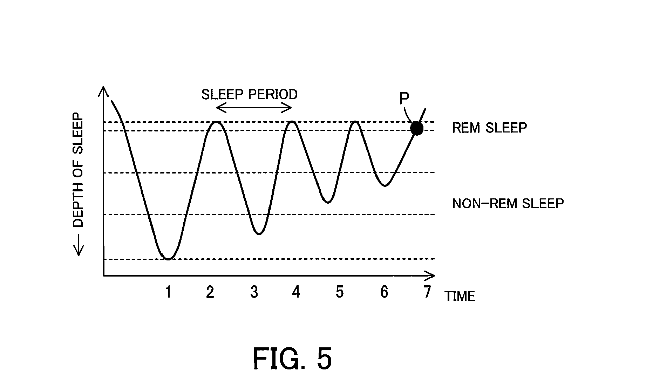

As shown in FIG. 5, the sleep information includes a sleep period and a sleep time. During sleep, REM sleep in which sleep is light and non-REM sleep in which sleep is deep are repeated at a specific period, and a time between the REM sleep and the REM sleep (or between the non-REM sleep and the non-REM sleep) is the sleep period. The state of the REM sleep is a timing P appropriate for waking up.

If the analysis part 16 calculates, based on the sleep information and the daily activity amount information for the past one month stored in the storage part 15, that for example, the daily activity amount is large when the sleep period of the user is one and half hour and the sleep time is seven and half hours, the analysis part calculates, based on the sleep period and the sleep time, that the appropriate hypnagogic time is "23:30" when the waking-up time is "7:00".

Further, by reverse calculation from the hypnagogic time "23:30", appropriate toning and dimming start time of illumination is "23:00", bathing end time is "22:30", television watching end time is "22:00", and meal end time is "21 :30".

With respect to the toning and dimming of illumination, an excessively bright light or a light including a lot of blue light hinders falling asleep. Thus, the illumination light is automatically adjusted to an appropriate state before sleeping and before the appropriate hypnagogic time by a specified time, so that falling asleep is not hindered.

With respect to bathing, if a bath is taken immediately before going to sleep, depth body-temperature is not lowered, and sleeping becomes difficult. Thus, bathing is urged to be ended before the appropriate hypnagogic time by specified time, so that falling asleep is not hindered.

With respect to television watching, similarly to the illumination, light emitted by the television may also prevent falling asleep. Thus, the television watching is urged to be ended before the appropriate hypnagogic time by specified time, so that falling asleep is not hindered.

With respect to a meal, if the meal is taken immediately before going to sleep, nerves and blood concentrate on digestion, and sleeping may become difficult, and stomach oppression may occur next morning. Thus, the meal is urged to be taken so that the meal is ended before the appropriate hypnagogic time by specified time, and falling asleep is not hindered.

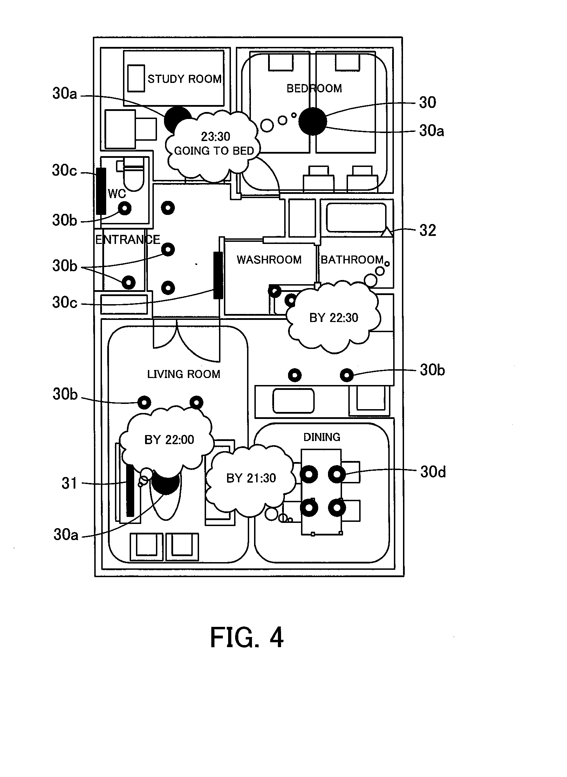

When the user returns home at "20:00", and the user accesses the server 23 by the terminal 13, the server 23 transmits to the terminal 13 such schedule information that the recommended hypnagogic time of today is "23:30", the meal time is "till 21:30", the television watching time is "till 22:00", and the bathing time is "till 22:30". As shown in FIG. 4, the terminal 13 receiving the schedule information displays the schedule information on the screen of the terminal 13, so that the user can confirm. As shown in FIG. 4, the in-house layout is displayed, and the time is displayed on the layout, so that the schedule information can be made easy to be confirmed. In FIG. 4, reference numeral 30 denotes luminaires (sealing light 30a, downlight 30b, line illumination 30c, dining light 30d), 31 denotes a television, and 32 denotes a bath water heater.

Incidentally, the schedule information to be displayed on the terminal 13 is not limited to the layout information, and may be displayed by characters or may be displayed together with figures and photographs.

The user who confirms the schedule information finishes the meal in the dining by "21:30". When the time becomes "21:30", the dining light is automatically turned off by the control of the server 23 or the home gateway 20. By this, the user can be urged to finish the meal by "21 :30".

The user can watch the television in the living room till "22:00". Even if the user continues to watch the television, when the time becomes "22:00", the television is automatically turned off by the control of the server 23 or the home gateway 20. By this, the user can be urged to finish the television watching by "22:00".

The server 23 or the home gateway 20 controls the bath water heater 32, and automatically heats the bath so that the user can take a bath at, for example, "22:00". The user takes the bath and finishes bathing by "22:30".

When the time becomes "23:00", the in-house luminaire emits bulb color illumination light with a small ratio of blue light by the control of the server 23 or the home gateway 20, and the illuminance is gradually lowered. By this, the user naturally feels sleepy and is urged to go to bed.

When the time becomes "23:30", all the illumination in the house is turned off by the control of the server 23 or the home gateway 20. By this, the user naturally falls asleep.

Besides, on next day, when the time becomes slightly before "7:00", the sealing light in the bedroom is turned on by the control of the server 23 or the home gateway 20, and waking up is naturally urged by the light. Further, when the time becomes "7:00", an alarm may be rung by the terminal 13, such as a smartphone, by the control of the server 23 or the home gateway 20.

As described above, since the in-house electric equipments 11 are automatically controlled based on the stored biological information of the user, the user can wake up in the fresh best state, and further, the healthy life in which the user can vigorously act in daytime can be expected to be urged.

Further, the user wears the biological sensor 14 and lives, and merely sets his/her own schedule, so that the electric equipments 11 are automatically operated, and the user is urged to have a healthy life.

Further, the appropriate hypnagogic time, which is difficult even for the user to be aware of, can be automatically calculated based on the wake-up scheduled time on next day and the past sleep information.

Similarly to the illumination, the light emitted by the television can also prevent sleeping. Thus, the television is automatically turned off before the appropriate hypnagogic time by the specified time, so that falling asleep is not hindered.

The excessively bright light or the light including a lot of blue light is known to prevent falling asleep. Thus, the illumination light is automatically controlled into the appropriate state before sleeping and before the appropriate hypnagogic time by the specified time, so that falling asleep can be made not to be hindered.

If a bath is taken immediately before sleeping, the depth body-temperature is not lowered and sleeping becomes difficult. Thus, the bath water heater operates so as to complete bathing before the appropriate hypnagogic time by the specified time and urges bathing, so that falling asleep can be made not to be hindered.

If a meal is taken immediately before going to sleep, nerves and blood concentrate on digestion, and sleeping may become difficult, and stomach oppression may occur next morning. Thus, the dining light is turned off at the specified time so that the meal is finished before the appropriate hypnagogic time by the specified time, and falling asleep can be made not to be hindered.

The lighting control of the luminaire is performed correspondingly to the wake-up time, so that the user can be urged to naturally wake up.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the spirit of the inventions. The accompanying claims and their equivalents are intended to cover such forms or modifications as would fall within the scope and spirit of the inventions.

## Claims

1. An in-house control system (10) for controlling an in-house electric equipment (11), comprising:
an acquisition unit (14) to acquire biological information of a user;
a storage part (15) to store the biological information acquired by the acquisition unit (14);
a setting part (13) to set schedule information by the user; and
a control part (12) to control the electric equipment (11) based on the schedule information set by the setting part (13) and the biological information stored in the storage part (15).

2. The system (10) according to claim 1, wherein
the setting part (13) sets wake-up time as the schedule information, and
the control part (12) determines appropriate hypnagogic time based on the wake-up time and sleep information of the user included in the biological information stored in the storage part (15), and controls the electric equipment (11) correspondingly to the hypnagogic time.

3. The system (10) according to claim 1, wherein
the setting part (13) sets wake-up time as the schedule information, and
the control part (12) determines a sleep period based on sleep information of the user included in the biological information stored in the storage part (15), determines a sleep time in which a daily activity amount of the user included in the biological information stored in the storage part (15) is large, determines appropriate hypnagogic time for the wake-up time based on the sleep period and the sleep time, and controls the electric equipment (11) correspondingly to the hypnagogic time.

4. The system (10) according to claim 2 or 3, wherein
the electric equipment (11) is a luminaire (30) to irradiate light, and
the control part (12) controls to decrease a ratio of blue light included in the light irradiated by the luminaire (30) correspondingly to the hypnagogic time, and controls to lower illuminance.

5. The system (10) according to any one of claims 1 to 3, wherein the control part (12) controls the electric equipment (11) correspondingly to wake-up time.

6. The system (10) according to claim 5, wherein
the electric equipment (11) is a luminaire (30) to irradiate light, and
the control part (12) performs lighting control of the luminaire (30) correspondingly to the wake-up time.

7. The system (10) according to any one of claims 1 to 6, further comprising a terminal (13) to display schedule information based on which the control part (12) controls the electric equipment (11).
